(19) Europäisches Patentamt
European Patent Office
Office européen des brevets

(11) Publication number: 0 179 239
A2

# (12) EUROPEAN PATENT APPLICATION

(21) Application number: 85111049.4

(22) Date of filing: 02.09.85

(51) Int. Cl.[4]: C 07 D 401/04
C 07 D 213/26, A 61 K 31/47
//C07D211/90, C07D213/00,
C07D213/38, C07D491/052,
(C07D401/04, 215:00, 213:00)

(30) Priority: 17.09.84 US 651121
17.09.84 US 650946

(43) Date of publication of application:
30.04.86 Bulletin 86/18

(84) Designated Contracting States:
AT BE CH DE FR GB IT LI NL SE

(71) Applicant: STERLING DRUG INC.
90 Park Avenue
New York New York(US)

(72) Inventor: Lesher, George Yohe
R.D. 1, Box 268 Miller Road
East Greenbush New York(US)

(72) Inventor: Daum, Sol Jacob
468 West Lawrence Street
Albany New York(US)

(72) Inventor: Baldev, Singh
3 Mountain Trail
East Greenbush New York(US)

(74) Representative: Baillie, Iain Cameron et al,
c/o Ladas & Parry Isartorplatz 5
D-8000 München 2(DE)

## (54) 7-(Pyridinyl)-/-alkyl-1,4-dihydro-4-oxo-3-quinolinecarboxylic acid having antibacterial activity and preparation thereof.

(57) 1-Ethyl-6-fluoro-1,4-dihydro-7-(2,6-dimethyl-4-pyridinyl)-4-oxo-3-quinolinecarboxylic acid or salt thereof, a novel compound useful highly potent antibacterial agent with a broad spectrum of anti-microbial activity, is prepared by nitrating the corresponding 6-desfluoro compound to produce the corresponding 6-nitro compound, reducing the latter compound to produce the corresponding 6-amino compound and converting the 6-amino via its diazonium salt to said 6-fluoro compound. Said novel acid is also prepared via intermediates prepared by heating 4-(2-fluorophenyl)-2,6-dimethyl-3,5-pyridinedicarboxylic acid to produce a mixture of 4-(2-fluorophenyl)-2,6-dimethylpyridine and 2,4-dimethyl-5H-[1]benzopyrano[3,4-c]pyridin-5-one, separating the components of said mixture and nitrating 4-(2-fluorophenyl)-2,6-dimethylpyridine to produce 4-(2-fluoro-5-nitrophenyl)-2,6-dimethylpyridine.

EP 0 179 239 A2

0179239

This invention relates to a novel 7-(pyridinyl)-1-alkyl-1,4-dihydro-4-oxo-3-quinolinecarboxylic acid, salts thereof and its preparation, said compound having antibacterial use.

British Patent 822,586, shows the nitration of 1-methyl-4-quinolone-3-carboxylic acid, alternatively named 1,4-dihydro-1-methyl-4-oxo-3-quinolinecarboxylic acid, by reacting it at room temperature with a nitrating agent comprising anhydrous nitric acid together with another anhydrous mineral acid, for example, sulfuric or phosphoric acid, to produce 1-methyl-6-nitro-4-quinolone-3-carboxylic acid, alternatively named 1,4-dihydro-1-methyl-6-nitro-4-oxo-3-quinolinecarboxylic acid.

British Patent 830,832 shows as antibacterial agents 1-alkyl-4-quinolone-3-carboxylic acids. Illustrative of compounds disclosed are 1-ethyl-6-nitro-4-quinolone-3-carboxylic acid (Example 38), which was prepared by nitrating 1-ethyl-4-quinolone-3-carboxylic acid at room temperature with a mixture of concentrated nitric acid and concentrated sulfuric acid and 1-ethyl-6-fluoro-4-quinolone-3-carboxylic acid (Example 17), alternatively named 1-ethyl-1,4-dihydro-6-fluoro-4-oxo-3-quinolinecarboxylic acid, which was prepared by heating 3-ethoxycarbonyl-6-fluoro-4-hydroxyquinoline with diethyl sulfate in aqueous sodium hydroxide solution.

U.S. Patent 3,753,993 shows as antibacterial agents 1-alkyl-1,4-dihydro-4-oxo-7-(pyridinyl)-3-quinolinecarboxylic acids. Illustrative of these compounds is 1-ethyl-1,4-dihydro-7-(2,6-dimethyl-4-pyridinyl)-4-oxo-3-quinolinecarboxylic acid (Example 6A, also known as Win 35,439), which was prepared stepwise as follows: first reacting 4-(3-aminophenyl)-2,6-dimethylpyridine with diethyl ethoxymethylenemalonate to produce diethyl 3-(2,6-dimethyl-4-pyridinyl)anilinomethylenemalonate (Example 6B), next heating the latter in an eutectic mixture of diphenyl and diphenyl ether (Dowtherm A) to produce ethyl 1,4-dihydro-7-(2,6-dimethyl-4-pyridinyl)-3-quinolinecarboxylate (Example 6C) and then heating said ester with ethyl iodide in dimethylformamide in the presence of anhydrous potassium carbonate to produce the ethyl ester of 1-ethyl-1,4-dihydro-7-(2,6-dimethyl-4-pyridinyl)-4-oxo-3-quinolinecarboxylic acid (Example 6A), which is hydrolyzed to produce the corresponding acid. Also shown in this patent as Example 1A is 1-ethyl-1,4-dihydro-4-oxo-7-(4-pyridinyl)-3-quinolinecarboxylic acid, now known generically as rosoxacin and also as Win 35,213.

U.S. Patent 3,907,808 shows as antibacterial agents

1-alkyl-1,4-dihydro-4-oxo-5(or 6)-(halo, lower-alkyl or lower-alkoxy)-7-(pyridinyl)-3-quinolinecarboxylic acids. Illustrative of these compounds is 7-(3,5-dicarboxy-2,6-dimethyl-4-pyridinyl)-1-ethyl-6-fluoro-1,4-dihydro-4-oxo-3-quinolinecarboxylic acid (Example 57A), which was prepared in six steps starting with 2-fluoro-5-nitrobenzaldehyde as follows: 1) a mixture containing 2-fluoro-5-nitrobenzaldehyde, methyl acetoacetate, methanol and concentrated ammonium hydroxide was refluxed to produce dimethyl 4-(2-fluoro-5-nitrophenyl)-1,4-dihydro-2,6-dimethyl-3,5-pyridinedicarboxylate (Example 57B); 2) oxidizing the product of Example 57B by heating it with 4N nitric acid to produce dimethyl 4-(2-fluoro-5-nitrophenyl)-2,6-dimethyl-3,5-pyridinedicarboxylate (Example 57C); 3) catalytically hydrogenating Example 57C to produce dimethyl 4-(5-amino-2-fluorophenyl)-2,6-dimethyl-3,5-pyridinedicarboxylate (Example 57D); 4) reacting Example 57D with diethyl ethoxymethylenemalonate to produce 3-(3,5-dicarbomethoxy)-2,6-dimethyl-4-pyridinyl)-4-fluoroanilinomethylenemalonate (Example 57E); 5) heating Example 57E in Dowtherm A to produce ethyl 7-(3,5-dicarbomethoxy-2,6-dimethyl-4-pyridinyl)-6-fluoro-1,4-dihydro-4-oxo-3-quinolinecarboxylate (Example 57F); and, 6) heating Example 57F with ethyl iodide in dimethylformamide in the presence of anhydrous potassium carbonate and saponifying the resulting compound to produce 7-(3,5-dicarboxy-2,6-dimethyl-4-pyridinyl)-1-ethyl-6-fluoro-1,4-dihydro-4-oxo-3-quinolinecarboxylic acid (Example 57A). Example

56F of this patent shows the conversion of 4-(2-methoxy-5-nitrophenyl)-2,6-dimethyl-3,5-pyridinedicarboxylic acid to 4-(2-methoxy-5-nitrophenyl)-2,6-dimethylpyridine by heating it in Dowtherm A. Also, Example 64C shows the conversion of 7-(3,5-dicarboxy-2,6-dimethyl-4-pyridinyl)-1-ethyl-1,4-dihydro-6-methyl-4-oxo-3-quinolinecarboxylic acid to 7-(2,6-dimethyl-4-pyridinyl)-1-ethyl-1,4-dihydro-6-methyl-4-oxo-3-quinolinecarboxylic acid by heating it in diethyl phthalate at 240-255°C.

U.S. Patent 4,146,719 shows as an antibacterial agent 1-ethyl-6-fluoro-1,4-dihydro-4-oxo-7-(1-piperazinyl)-3-quinolinecarboxylic acid, now known as norfloxacin, and its salts.

U.S. Patent 4,292,317 shows as antibacterial agents 6-halo-1-substituted-7-substituted-amino-1,4-dihydro-4-oxo-3-quinolinecarboxylic acids and salts thereof, including 1-ethyl-6-fluoro-1,4-dihydro-7-(4-methyl-1-piperazinyl)-4-oxo-3-quinolinecarboxylic acid, now known as pefloxacin.

The present invention resides in 1-ethyl-6-fluoro-1,4-dihydro-4-oxo-7-(2,6-dimethyl-4-pyridinyl)-3-quinolinecarboxylic acids of the formula I.

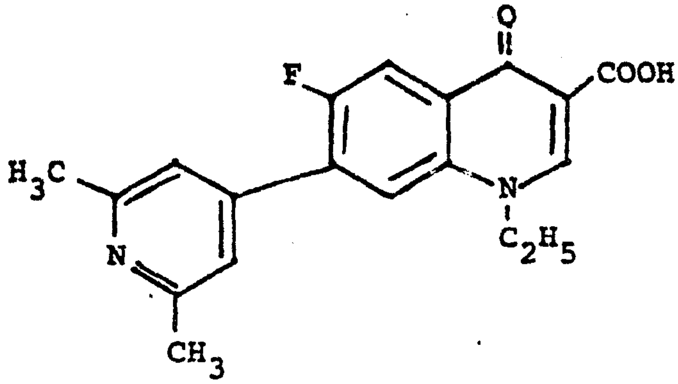

I

and pharmaceutically acceptable, non-toxic acid-addition or cationic salts thereof. The compound of formula I and its said salts are useful as antibacterial agents, as determined by standard bacteriological evaluation procedures.

Preferably the compound of formula I is in free base form. As shown hereinbelow, this compound is a highly potent antibacterial agent having a wide spectrum of activity against a wide variety of microorganisms, including anaerobic microorganisms associated with periodontal diseases.

A composition for combatting bacteria comprises an antibacterially effective amount of 1-ethyl-6-fluoro-1,4-dihydro-7-(2,6-dimethyl-4-pyridinyl)-4-oxo-3-quinolinecarboxylic acid of formula I or a pharmaceutically acceptable non-toxic acid-addition or cationic salt thereof together with one or more pharmaceutically acceptable vehicles or excipients. A method for combatting bacteria comprises contacting the locus of said bacteria with a composition containing the compound of formula I or pharmaceutically acceptable non-toxic acid-addition or cationic salt thereof.

One can produce the above compound of the invention by:

a. converting 6-amino-1-ethyl-1,4-dihydro-7-(2,6-dimethyl-4-pyridinyl)-4-oxo-3-quinolinecarboxylic acid via its 6-diazonium salt to produce 1-ethyl-6-fluoro-1,4-dihydro-7-(2,6-dimethyl-4-pyridinyl)-4-oxo-3-quinolinecarboxylic acid or

b. ethylating a 6-fluoro-1,4-dihydro-7-(2,6-dimethyl-4-pyridinyl)-4-oxo-3-quinolinecarboxylic acid or lower-alkyl ester thereof and in the case of the reaction with the lower alkyl ester thereby producing a lower-alkyl ester of 1-ethyl-6-fluoro-1,4-dihydro-7-(2,6-dimethyl-4-pyridinyl)-4-oxo-3-quinolinecarboxylic acid, hydrolyzing said ester to produce the corresponding acid,

and, if desired, converting a free base obtained to an acid-addition salt thereof or converting a compound obtained to a cationic salt thereof by reaction with a strong inorganic or organic base. The complete sequence according to process a) comprises nitrating 1-ethyl-1,4-dihydro-7-(2,6-dimethyl-4-pyridinyl)-4-oxo-3-quinolinecarboxylic acid to produce 1-ethyl-1,4-dihydro-7-(2,6-dimethyl-4-pyridinyl)-6-nitro-4-oxo-3-quinolinecarboxylic acid, reducing said 6-nitro compound to produce 6-amino-1-ethyl-1,4-dihydro-7-(2,6-dimethyl-4-pyridinyl)-4-oxo-3-quinolinecarboxylic acid and converting said 6-amino compound via its 6-diazonium salt

to produce said 1-ethyl-6-fluoro-1,4-dihydro-7-(2,6-dimethyl-4-pyridinyl)-4-oxo-3-quinolinecarboxylic acid. In the reduction step, the preferred reducing agent is sodium hydrosulfite in an aqueous alkaline medium.

A process for purifying 1-ethyl-6-fluoro-1,4-dihydro-7-(2,6-dimethyl-4-pyridinyl)-4-oxo-3-quinolinecarboxylic acid containing as impurities the corresponding 6-desfluoro and 6-hydroxy compounds comprises converting it to its corresponding lower-alkyl 3-carboxylate, separating the ester from the reaction mixture and saponifying the ester to produce the purified acid.

In the conversion of the corresponding 6-amino compound to the 6-fluoro compound of formula I, the conversion is preferably carried out by first preparing the 6-diazonium chloride, reacting it with hexafluorophosphoric acid, i.e., $HPF_6$, to produce the corresponding 6-diazonium hexafluorophosphate, conveniently as its hydrofluorophosphate, and heated the latter to produce the 6-fluoro compound of formula I.

In the purification process aspect of the invention, the ethyl 3-carboxylate is formed, isolated and saponified to produce the purified compound of formula I.

The preferred process, according to process b), comprises heating 4-(2-fluorophenyl)-2,6-dimethyl-3,5-pyridinedicarboxylic acid to produce a mixture of 4-(2-fluorophenyl)-2,6-dimethylpyridine and 2,4-dimethyl-5H-[1]benzopyrano[3,4-c]pyridin-5-one, separating the components of said mixture, and nitrating the 4-(2-fluorophenyl)-2,6-dimethylpyridine thus obtained to produce 4-(2-fluoro-5-nitrophenyl)-2,6-dimethylpyridine. As shown hereinbelow, 4-(2-fluoro-5-nitrophenyl)-2,6-dimethylpyridine is useful as an intermediate to prepare in very good yield the corresponding 4-(5-amino-2-fluorophenyl)-2,6-dimethylpyridine which is converted in four steps (all in very good yields) to 1-ethyl-6-fluoro-1,4-dihydro-7-(2,6-dimethyl-4-pyridinyl)-4-oxo-3-quinolinecarboxylic acid. The overall process provides an improved and convenient means for preparing large scale quantities of said antibacterial agent.

The invention further resides in a compound of the formula II

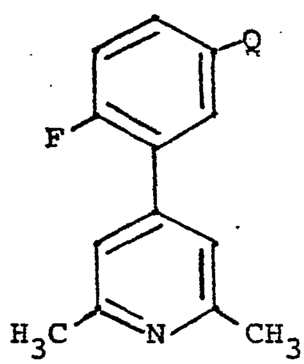

II

where Q is hydrogen or nitro, or acid-addition salt thereof. As noted above, these compounds are useful as key intermediates in the preparation of the highly potent antibacterial agent, 1-ethyl-6-fluoro-1,4-dihydro-7-(2,6-dimethyl-4-pyridinyl)-4-oxo-3-quinolinecarboxylic acid.

The compounds of formulas I and II are useful both in the free base form and in the form of acid-addition salts, and, both forms are within the purview of the invention. The acid-addition salt is simply an alternative form for use; and in practice, use of the salt form inherently amounts to use of the base form. The acids which can be used to prepare the acid-addition salts of the compounds of formulas I and II include preferably those which produce, when combined with the free base, pharmaceutically acceptable salts, that is, salts whose anions are relatively innocuous to the animal organism in pharmaceutical doses of the salts, so that the beneficial antibacterial properties inherent in the free base are not vitiated by side effects ascribable to the anions. Appropriate pharmaceutically acceptable salts within the scope of

the invention are those derived from mineral acids such as hydrochloric acid, hydrobromic acid, sulfuric acid, phosphoric acid and sulfamic acid; and organic acids such as lactic acid, acetic acid, citric acid, tartaric acid, methanesulfonic acid, ethanesulfonic acid, benzenesulfonic acid, p-toluenesulfonic acid, cyclohexylsulfamic acid, quinic acid, and the like, giving the hvrochloride, hydrobromide, sulfate, phosphate sulfamate, lactate, acetate, citrate, tartrate, methanesulfonate, ethanesulfonate, benzenesulfonate, p-toluenesulfonate, cyclohexylsulfamate and quinate, respectively.

The acid-addition salts of said compounds of formulas I and II are prepared either by dissolving the free base in aqueous or aqueous-alcohol solution or other suitable solvents containing the appropriate acid and isolating the salt by evaporating the solution, or by reacting the free base and acid in an organic solvent, in which case the salt separates directly or can be obtained by concentration of the solution.

Other pharmaceutically acceptable salts of said compound of formula I are those cationic salts derived from strong inorganic or organic bases, e.g., sodium hydroxide, potassium hydroxide, trimethylammonium hydroxide, to produce the corresponding cationic salt, e.g., sodium, potassium, trimethylammonium salt, respectively.

Although pharmaceutically acceptable salts of said basic compounds of formula II is preferred, all acid-addition salts are within the scope of the invention. All acid-addition salts are useful as sources of the free base form even if the particular salt *per se* is desired only as an intermediate product as for example when the salt is formed only for purposes of purification or identification, or when it is used as an intermediate in preparing a pharmaceutically acceptable salt by ion exchange procedures.

The molecular structure of the compounds of formula I and corresponding intermediate 6-nitro and 6-amino compounds and of the compounds of formula II were assigned on the basis of evidence provided by infrared, ultraviolet, nuclear magnetic resonance and mass spectra, by the correspondence of calculated and found values for the elemental analyses, and by their method of preparation.

The manner of making and using the instant invention will now be generally described so as to enable a person skilled in the art of pharmaceutical chemistry to make and use the same.

The nitration of the known 1-ethyl-1,4-dihydro-7-(2,6-dimethyl-4-pyridinyl)-4-oxo-3-quinolinecarboxylic acid (U.S. Patent 3,753,993) to produce the corresponding 6-nitro compound was carried out by reacting said known compound with concentrated nitric acid in the presence of concentrated sulfuric acid, the reaction carried out by first mixing the reactants at ambient temperature

and then heating the reaction mixture at about 90°C. to 110°C., conveniently on a steam bath. The product was obtained by pouring the cooled reaction mixture into excess ice water and partially neutralizing (to a pH of about 5.5 to 6.0) the aqueous acidic solution with concentrated ammonium hydroxide and collecting the precipitated 6-nitro compound.

The conversion of 1-ethyl-1,4-dihydro-7-(2,6-dimethyl-4-pyridinyl)-6-nitro-4-oxo-3-quinolinecarboxylic acid to the corresponding 6-amino compound was conveniently carried out by heating the 6-nitro compound at about 90°C. to 110°C., conveniently on a steam bath, with sodium hydrosulfite (also named sodium dithionite) in an aqueous alkaline medium containing an organic solvent, e.g., dimethylformamide, sodium bicarbonate conveniently used to make the solution alkaline. The 6-amino compound was isolated by acidifying the cooled reaction mixture to a pH of about 5.5, preferably with acetic acid, and collecting the precipitated 6-amino compound. Alternatively, the reduction of the 6-nitro compound to the corresponding 6-amino compound can be carried out by catalytic hydrogenation using palladium-on-charcoal as catalyst and acetic acid as solvent.

The conversion of 6-amino-1-ethyl-1,4-dihydro-7-(2,6-dimethyl-4-pyridinyl)-4-oxo-3-quinolinecarboxylic acid to the corresponding 6-fluoro compound was done by first preparing the 6-diazonium salt, preferably chloride, treating its aqueous solution at about -10°C. (using an ice-salt bath) with hexafluorophosphoric acid (carefully added in one portion), collecting the precipitated 6-diazonium hexafluorophosphate as its hydrohexafluorophosphate, heating the diazonium salt at about 200°C. to 240°C., preferably about 225°C. to 235°C., in a suitable solvent, e.g., paraffin oil, dissolving the cooled reaction mixture with aqueous ammonium hydroxide (e.g., 5N), filtering the solution and slightly acidifying (to a pH of about 5.5) the filtrate with acetic acid and collecting the precipitated 6-fluoro compound (of formula I), which contained about 10-15% of the 6-desfluoro compound (produced during decomposition of the diazonium salt) and a trace of the corresponding 6-hydroxy compound. The 6-fluoro compound was separated from said impurities by converting it to its lower-alkyl, preferably ethyl, ester, separating the ester from the corresponding ester of the 6-desfluoro compound and of the 6-hydroxy compound by high pressure liquid chromatography and then saponifying the ester to produce the purified 6-fluoro acid of formula I.

As shown hereinbelow in Example 3, the intermediate 4-(2-fluorophenyl)-2,6-dimethyl-3,5-pyridinedicarboxylic acid used in process b, was prepared in very good yield by first adding concentrated aqueous ammonia to a mixture of 2-fluorobenzaldehyde and methyl acetoacetate in methanol to produce dimethyl 4-(2-fluorophenyl)-1,4-dihyro-2,6-dimethyl-3,5-pyridinedicarboxylate, oxidizing the 1,4-dihydro compound with 4N nitric acid to produce dimethyl 4-(2-fluorophenyl)-2,6-dimethyl-3,5-pyridinedicarboxylate and saponifying the latter ester by heating it with sodium hydroxide in aqueous methanol at about 60-65°C. to produce 4-(2-fluorophenyl)-2,6-dimethyl-3,5-pyridinedicarboxylic acid.

Decarboxylation of 4-(2-fluorophenyl)-2,6-dimethyl-3,5-pyridinedicarboxylic acid to produce 4-(2-fluorophenyl)-2,6-dimethylpyridine was carried out conveniently by heating the dicarboxylic acid at about 240°C. to 300°C., preferably about 240-255°C., in a suitable high boiling solvent, preferably by refluxing said dicarboxylic acid in a eutectic mixture of diphenyl and diphenyl ester (Dowtherm A) whereupon there resulted in the formation of 4-(2-fluorophenyl)-2,6-dimethylpyridine and varying amounts of 2,4-dimethyl-5H-[1]benzopyrano[3,4-c]pyridin-5-one as a side product, filtering off the side product from the cooled reaction mixture and then isolating the desired product. After removal of the side product, the desired product is isolated from the remaining reaction mixture by extracting it with an aqueous mineral acid, preferably HCl, making the acidic extract alkaline, preferably with aqueous ammonia, and extracting the product with a suitable solvent such as chloroform, ether or methylene dichloride. Other high boiling solvents that can be used in place of Dowtherm A are mineral oil,

diethyl phthalate and the like. In using the preferred Dowtherm A, it was found that a greater proportion of the desired 4-(2-fluorophenyl)-2,6-dimethylpyridine and a lesser amount of the said side product were obtained by using a larger quantity of Dowtherm A. Use of recycled or previously used Dowtherm A decreased the yield of desired product.

The conversion of 4-(2-fluorophenyl)-2,6-dimethylpyridine to 4-(2-fluoro-5-nitrophenyl)-2,6-dimethylpyridine was carried out by adding a molar equivalent quantity or slight excess of a metal nitrate, preferably potassium nitrate, to 4-(2-fluorophenyl)-2,6-dimethylpyridine in excess concentrated sulfuric acid, keeping the reaction temperature below about 0°C., preferably between about -10°C. to -15°C., conveniently accomplished using an salt-ice bath. Alternatively, other alkali metal or alkaline earth metal nitrates, e.g., sodium nitrate or calcium nitrate, can be used in place of potassium nitrate.

As illustrated hereinbelow in Example 6, 4-(2-fluoro-5-nitrophenyl)-2,6-dimethylpyridine is converted to 1-ethyl-6-fluoro-1,4-dihydro-7-(2,6-dimethyl-4-pyridinyl)-4-oxo-3-quinolinecarboxylic acid in very good yield by first reducing 4-(2-fluoro-5-nitrophenyl)-2,6-dimethylpyridine by catalytic hydrogenation to produce 4-(5-amino-2-fluorophenyl)-2,6-dimethylpyridine, alternatively named 3-(2,6-dimethyl-4-pyridinyl)-4-fluorobenzene-

amine (Example 6A), reacting the 5-amino-2-fluorophenyl compound of (Example 6A) with a di-lower-alkyl (diethyl)ethoxymethylenemalonate (EMME) to produce a di-lower-alkyl (diethyl) 3-(2,6-dimethyl-4-pyridinyl)-4-fluoroanilinomethylenemalonate (Example 4B), refluxing the EMME adduct of Example 6B in Dowtherm A to produce a lower-alkyl (ethyl) 6-fluoro-1,4-dihydro-7-(2,6-dimethyl-4-pyridinyl)-4-oxo-3-quinolinecarboxylate (Example 6C), ethylating the compound of Example 6C by heating it with an ethylating agent, that is, the ethyl ester of an inorganic acid or a strong organic acid, e.g., ethyl iodide, in dimethylformamide in the presence of anhydrous potassium carbonate to produce ethyl 1-ethyl-6-fluoro-1,4-dihydro-7-(2,6-dimethyl-4-pyridinyl)-4-oxo-3-quinolinecarboxylate (Example 6D) and hydrolyzing the compound of Example 6D by refluxing it in aqueous sodium hydroxide solution containing methanol to produce 1-ethyl-6-fluoro-1,4-dihydro-7-(2,6-dimethyl-4-pyridinyl)-4-oxo-3-quinolinecarboxylic acid (Example 6E). Although the carboxylate is ethylated according to the above preferred procedure, it is also possible to hydrolyze the lower-alkyl ester obtained by Example 6C and ethylate the resulting acid under substantially the same conditions for said respective corrosion steps.

The following examples will further illustrate the invention without limiting it thereto.

Example 1

A. 1-Ethyl-1,4-dihydro-7-(2,6-dimethyl-4-pyridinyl)-6-nitro-4-oxo-3-quinolinecarboxylic Acid - To a stirred solution containing 200 g. of 7-(2,6-dimethyl-4-pyridinyl)-1-ethyl-1,4-dihydro-4-oxo-3-quinolinecarboxylic acid in 440 ml of 95-98% $H_2SO_4$ was added a solution of 80 ml of 90% $HNO_3$ in 80 ml of 95-98% $H_2SO_4$ over a period of one hour. The resulting solution was stirred at room temperature for an additional 20 minutes, then heated on a steam bath for 8 hours. The reaction mixture was allowed to stand at room temperature overnight and then poured into 6000 ml of ice water. The precipitate was collected and slurried in approximately 2000 ml water. Concentrated $NH_4OH$ was added to reach a pH of 5.5-6.0. The solid was collected and washed with a small amount of ethanol and ether. Additional material was obtained by treating the original filtrate with $NH_4OH$ as described above. Recrystallization from dimethylformamide gave several crops totalling 135 g (59%) and melting from 274°C. to 298°C. A 2.8 g sample was recrystallized from 20 ml of dimethylformamide, washed with ether and dried at 120°C. for 16 hours at 0.02 mm to produce 1.54 g of 1-ethyl-1,4-dihydro-7-(2,6-dimethyl-4-pyridinyl)-6-nitro-4-oxo-3-quinolinecarboxylic acid, m.p. 289-291°C.

B. 6-Amino-1-ethyl-1,4-dihydro-7-(2,6-dimethyl-4-pyridinyl)-4-oxo-3-quinolinecarboxylic Acid - A 35 g portion of 1-ethyl-1,4-dihydro-6-nitro-7-(2,6-dimethyl-4-pyridinyl)-4-oxo-3-quinolinecarboxylic acid was suspended in a mixture of 350 ml of hot dimethylformamide, 280 ml of saturated $NaHCO_3$ and 70 ml of water and the suspension was heated on a steam bath with stirring. A mixture of 58 g of sodium hydrosulfite in 100 ml of water was added in portions over a period of 20 minutes, during which time the reaction mixture became a clear solution and then darkened. More saturated $NaHCO_3$ solution (50 ml) was added and the reaction mixture was heated for 16 hours, after which time reduction was not complete. More sodium hydrosulfite (12 g) was added directly to the reaction mixture and heating continued for 1 hour after which time the reduction appeared to be complete (by TLC). Water (100 ml) was added, redissolving some of the material which had come out of solution; and, the mixture was heated an additional 90 minutes to insure completion of the reduction. A 1.15 g sample, m.p. 288-290°C. with decomposition, was recrystallized from ethanol and dried for 16 hours at 130°C. under high vacuum to yield 0.9 g of 6-amino-1-ethyl-1,4-dihydro-7-(2,6-dimethyl-4-pyridinyl)-4-oxo-3-quinolinecarboxylic acid as its monohydrate, m.p. >300°C.

C. <u>3-Carboxy-1-ethyl-1,4-dihydro-7-(2,6-dimethyl-4-pyridinyl)-4-oxo-6-quinolinyldiazonium Hexafluorophosphate</u> - A 20 g portion of 6-amino-1-ethyl-1,4-dihydro-7-(2,6-dimethyl-4-pyridinyl)-4-oxo-3-quinolinecarboxylic acid monohydrate was suspended in 100 ml of 6N HCl and

placed in an ice/salt bath. When the temperature of the mixture had reached -10°C., a cold solution of 4.5 g of $NaNO_2$ in 20 ml of $H_2O$ was added in portions over a period of a few minutes. The reaction mixture was stirred for 5-10 minutes during which time it became a clear solution. Hexafluorophosphoric acid (18 ml) was carefully added in one portion whereupon a precipitate separated immediately, the temperature rising to 0°C. before dropping again. The reaction mixture was stirred for 30 minutes after addition; and, then the precipitate was collected and washed successively with water, a small volume of cold ethanol, and finally with ether. The product was vacuum-dried at 50°C. for 48 hours in the presence of $P_2O_5$ to give 29.7 g (78%) of 3-carboxy-1-ethyl-1,4-dihydro-7-(2,6-dimethyl-4-pyridinyl)-4-oxo-6-quinolinyldiazonium hexafluorophosphate as its hydrohexafluorophosphate, m.p. 200-205°C. with decomposition.

D. <u>1-Ethyl-6-fluoro-1,4-dihydro-7-(2,6-dimethyl-4-pyridinyl)-4-oxo-3-quinolinecarboxylic Acid</u> - A 19.6 g portion of the diazonium salt of Example 3 was placed in a 3-necked round bottom flask equipped with a mechanical stirrer and connected to a nitrogen apparatus. Paraffin oil (200 ml) was added, the apparatus evacuated and filled with nitrogen. The reaction vessel was then immersed in an oil bath preheated to 230°C. After 10 minutes the starting material gummed up and formed a

ball, making stirring difficult. After heating for a total of 25 minutes the hot oil was decanted and the reaction mixture allowed to cool. It was then washed with n-hexane, broken up with a pestle and dissolved in 5N $NH_4OH$ (approximately 900 ml). This solution was filtered through a pad of diatomaceous earth and the filtrate acidified to a pH of 5.5 with concentrated acetic acid. After being refrigerated overnight, the precipitate (the 6-fluoro compound) was collected and found to contain a trace of the corresponding 6-hydroxy compound (evidence MS) and 10-15% of the correspondng 6-desfluoro compound (evidence NMR). Recrystallization from dimethylformamide yielded a first crop of 4.4 g (43%) and a second crop of 1.5 g (15%). Products from several runs were combined and after two recrystallizations from DMF still contained approximately 10% of the 6-desfluoro compound. This compound was ultimately purified (Example 2) by converting it to the ethyl ester, separating it from the 6-desfluoro ethyl ester by high pressure liquid chromatography, then saponifying it back to the acid.

Example 2

A. Ethyl 1-Ethyl-6-fluoro-1,4-dihydro-7-(2,6-dimethyl-4-pyridinyl)-4-oxo-3-quinolinecarboxylate - A 12.3 g sample of the 6-fluoro compound containing about 10% of the 6-desfluoro compound (i.e., the final product of Example 1D) and another 12.2 g of the same 6-fluoro

compound obtained from mother liquors of other runs and of varying purity were converted to 14.8 g of ethvl ester by refluxing for 48 hours in 1N ethanolic HCl containing a small quantity of triethyl orthoformate to take up any traces of water present (In a typical example 600 ml 1N ethanolic HCl and 3 ml triethyl orthoformate were used for 5.0 g of fluoro acid). The 14.8 g of ester was purified by preparative HPLC using a solvent system of 60% acetone/40% hexane to give 8.3 g of pure 6-fluoro ester, i.e., ethyl 1-ethyl-6-fluoro-1,4-dihvdro-7-(2,6-dimethyl-4-pyridinyl)-4-oxo-3-quinolinecarboxylate, which was used directly in the next step (Example 2A).

B. 1-Ethyl-6-fluoro-1,4-dihydro-7-(2,6-dimethyl-4-pyridinyl)-4-oxo-3-quinolinecarboxylic Acid - To 8.39 of the ethyl ester of Example 2A was added 200 ml of water and 1.8 g of sodium hydroxide. The mixture was heated on a steam bath for 3 and 1/2 hours, after which hydrolysis was incomplete. NaOH (0.9 g) was added and heating continued for 16 hours. The hot reaction mixture was filtered and the filtrate allowed to cool, after which it was acifified to a pH of 5-5.5 with concentrated acetic acid. The precipitate was collected and washed successively with water, ethanol and ether. The product was recrystallized from dimethylformamide to yield 6.7 g (88%) of 1-ethyl-6-fluoro-1,4-dihydro-7-(2,6-dimethyl-4-pyridinyl)-4-oxo-3-qunolinecarboxylic acid, m.p. >300°C.

## Example 3

A. Dimethyl 4-(2-fluorophenyl)-1,4-dihydro-2,6-dimethyl-3,5-pyridinedicarboxylate - To a stirred solution containing 119 g of 2-fluorobenzaldehyde, 600 ml of methanol and 255 ml of methyl acetoacetate was added 200 ml of concentrated ammonium hydroxide whereupon an exothermic reaction ensued. The reaction mixture was stirred at ambient temperature for 30 minutes, next refluxed a few hours and then allowed to stand at room temperature overnight (about 15 hours). The yellow solid that crystallized was collected, washed with ether and dried in an oven at 50-60°C. to yield 201 g of dimethyl 4-(2-fluorophenyl)-1,4-dihydro-2,6-dimethyl-3,5-pyridinedicarboxylate, m.p. 206-208°C. Concentration of the mother liquor yielded another 26.4 g of the product, m.p. 204-206°C. The total yield was 74%.

B. Dimethyl 4-(2-fluorophenyl)-2,6-dimethyl-3,5-pyridinedicarboxylate - To 600 ml of 90% nitric acid heated to about 70°C. was added with stirring over a period of 20 minutes 302.9 g of dimethyl 4-(2-fluorophenyl)-1,4-dihydro-2,6-dimethyl-3,5-pyridinedicarboxylate. The reaction mixture was heated on a steam bath for 1 hour and then allowed to cool with stirring for 2 hours. The reaction solution was then chilled and made basic with ammonium hydroxide solution. The resulting precipitate was collected, washed with water and dried in a

vacuum oven at 75°C. to yield 302.8 g of yellow solid. The solid was slurried in water, collected, washed with water and dried in a vacuum oven at 65°C. to yield 273.7 g (90%) of product, m.p. 85-88°C., which was used directly in the next step (Example 1C). A 20 g sample of the product was dried at 70°C., recrystallized from n-hexane and dried in a vacuum oven at 65°C. to yield 15.9 g of dimethyl 4-(2-fluorophenyl)-2,6-dimethyl-3,5-pyridinedicarboxylate, m.p. 94-96°C.

C. <u>4-(2-Fluorophenyl)-2,6-dimethyl-3,5-pyridinedicarboxylic Acid</u> A mixture containing 69 g of dimethyl 4-(2-fluorophenyl)-2,6-dimethyl-3,5-pyridinedicarboxylate, 300 ml of methanol and 100 ml of 35% aqueous sodium hydroxide solution was refluxed with stirring for 5 hours and then allowed to stand at room temperature overnight. The methanol was removed using a rotary evaporator and to the residual liquid was added 200 ml of water and filtered. The filtrate was acidifed with concentrated hydrochloric acid whereupon there precipitated a cream colored solid, which was washed with water and dried at 80-85°C. to yield 52.6 g (88%) of 4-(2-fluorophenyl)-2,6-dimethyl-3,5-pyridinedicarboxylic acid, m.p. >285°C.

<u>Example 4</u>

<u>4-(2-Fluorophenyl)-2,6-dimethylpyridine</u> - To 1400 ml of Dowtherm A heated to 240-245°C. was added over a 10 minute period 113 g of 4-(2-fluorophenyl)-2,6-dimethyl-3,5-pyridinedicarboxylic acid and the reaction mixture was boiled for 50 minutes, cooled and allowed to stand at room temperature overnight. The crystalline solid, a by-product identified below, was filtered off and the filtrate was extracted with 700 ml of 3N hydrochloric acid followed by a second extraction with 300 ml of 3N hydrochloric acid. The combined acidic extracts were made basic by adding aqueous ammonium hydroxide and the alkaline mixture was extracted with 800 ml of chloroform. Evaporation of the chloroform yielded a brown mixture of solid and liquid, to which was added 300 ml of n-hexane and the mixture chilled. The solid was filtered off and dried. This solid, a by-product, was combined with the above noted solid by-product to yield 25.4 g (29%) of 2,4-dimethyl-5H-[1]benzopyrano[3,4-c]pyridin-5-one, m.p. 194-197°C. The filtrate was concentrated to dryness on a steam bath, the residue taken up with ethanol and the solution concentrated on a rotary evaporator to yield, as a liquid, 55.2 g (70%) of 4-(2-fluorophenyl)-2,6-dimethylpyridine.

In another run as in the preceding paragraph using 113 g of 2-(2-fluorophenyl)-2,6-dimethyl-3,5-pyridinedicarboxylic acid, 1.1 liter of recovered Dowtherm A (used in a previous run) filtered through diatomaceous earth plus 300 ml of fresh of Dowtherm A, there was obtained 28.4 g (32%) of by-product, 2,4-dimethyl-5H-[1]benzo-

pyrano[3,4-c]pyridin-5-one, m.p. 193-196°C. and 44.8 g (57%) of 4-(2-fluorophenyl)-2,6-dimethylpyridine in free base form as a liquid.

In another run using a smaller proportionate amount of Dowtherm A, that is, 1200 ml, and 200 g of 4-(2-fluorophenyl-2,6-dimethyl-3,5-pyridinedicarboxylic acid. there was obtained a greater proportionate amount of said by-product, namely 57 g (36.7%) of 2,4-dimethyl-5H-[1]benzopyrano[3,4-c]pyridin-5-one and 42 g (30%) of 4-(2-fluorophenyl)-2,6-dimethylpyridine in free base form.

The above three batches of by-product, were combined, recrystallized from methanol and dried at 70-75°C. to yield 141 g of 2,4-dimethyl-5H-[1]benzopyrano[,4-c]-pyridin-5-one, m.p. 204-207°C.

The above three batches of 4-(2-fluorophenyl)-2,6-dimethylpyridine were combined and mixed well with 400 ml of n-hexane and 600 ml of 3N aqueous hydrochloric acid. The aqueous layer was separated, treated with decolorizing charcoal and concentrated to dryness on a rotary evaporator to give a white solid residue, about 9 g of which was recrystallized from isopropyl alcohol and dried over $P_2O_5$ at room temperature to yield 7.8 g of 4-(2-fluorophenyl)-2,6-dimethylpyridine hydrochloride hemihydrate, m.p. 200-203°C. The remainder of the solid residue was dissolved in 700 ml of water, the solution made basic with aqueous ammonium hydroxide and the

resulting alkaline solution extracted twice with 500 ml portions of chloroform. Removal of the chloroform yielded 148 g of 4-(2-fluorophenyl)-2,6-dimethylpyridine in free base form as a light brown liquid, which was used in the next step (Example 5).

Example 5

4-(2-fluoro-5-nitrophenyl-2,6-dimethylpyridine - To 55 ml of concentrated sulphuric acid cooled in a salt-ice bath to about -10 to -15°C. was added 10.1 g of potassium nitrate over a 5 minute period. The mixture was allowed to stir for 10 minutes and then to it was added dropwise over a 30 minute period 18.5 g of 4-(2-fluorophenyl)-2,6-dimethylpyridine whereupon a gummy solid separated and a viscous slurry was obtained, the reaction temperature rising to about 0°C. In order to facilitate stirring, another 50 ml portion of concentrated sulphuric acid was added dropwise over a 15 minute period and the resulting mixture was stirred in the salt-ice bath and then stirred for a period of about 5 hours while allowing the temperature of the reaction mixture to rise to room temperature. The reaction mixture was poured onto a large excess of ice and the mixture was neutralized by adding aqueous ammonium hydroxide solution. The resulting light yellow fluffy precipitate was collected, washed with water and air dried. The solid was then dissolved in 300 ml of chloroform, the solution treated with decolorizing charcoal and filtered. The filtrate was concentrated on a rotary evaporator and the residue was slurred with 200 ml of 1:1 ether-n-hexane, the mixture filtered and the collected solid dried at 70-75°C. to yield 18.67 g (84%) of 4-(2-fluoro-5-nitrophenyl)-2,6-dimethylpyridine, m.p. 169-171°C.

Example 6

A. 3-(2,6-dimethyl-4-pyridinyl)-4-fluorobenzeneamine - 4-(2-fluoro-5-nitrophenyl)-2,6-dimethylpyridine, 180 g, was divided into four equal portions and each portion was reduced separately as follows. A mixture containing 45 g of 4-(2-fluoro-5-nitrophenyl)-2,6-dimethylpyridine, 200 ml of acetic acid and 800 mg of platinum dioxide was catalytically hydrogenated until hydrogen uptake ceased, about 4 hours with little hydrogen uptake the last thirty minutes. The catalyst was filtered off, washed with 300 ml of water, and the filtrate made basic by adding aqueous ammonium hydroxide. The solution was cooled to yield a oily material which solidified. The solid was collected, washed with water and dried to yield the desired amine. The remaining three 45 g portions of 4-(2-fluoro-5-nitrophenyl)-2,6-dimethylpyridine were reduced in the same way and the combined amine products were dissolved in 600 ml of concentrated hydrochloric acid and heated on a steam bath with stirring for 4 and 1/2 hours and then treated with decolorizing charcoal

and filtered. The filtrate was made basic by adding aqueous ammonia and the resulting mixture was chilled to yield an oil which crystallized. The crystalline material was collected, washed with water and dried in an oven to yield 164.9 g of gummy solid. The solid was dissolved in ether containing 5% methanol and the solution was filtered through silica gel to remove the gummy impurities. The ether-methanol solvent of the filtrate was distilled off _in vacuo_ and the residue was recrystallized from ether-isopropyl alcohol to yield 80.9 g of 3-(2,6-dimethyl-4-pyridinyl)-4-fluorobenzeneamine, m.p. 142-145°C. Concentration of the mother liquor yielded another 43.4 g of the product, m.p. 141-144°C., thereby giving a total yield of 82%.

B. Diethyl 4-fluoro-3-(2,6-dimethyl-4-pyridinyl)anilinomethylenemalonate - A mixture containing 122 g of 3-(2,6-dimethyl-4-pyridinyl)-4-fluorobenzeneamine, 130 g of diethyl ethoxymethylenemalonate and 120 ml of toluene was heated with stirring at 130-135°C. for 3 and 1/2 hours using an air condenser allowing ethanol to escape from the reaction mixture. The reaction mixture was cooled and dissolved in one liter of boiling n-hexane. The hot solution was filtered through diatomaceous earth, the pad washed with hot n-hexane (400 ml), and the combined filtrate and washings were allowed to stand at

room temperature for 18 hours. The white crystalline solid that separated was collected, washed with n-hexane and dried at 60-65°C. to yield 159.6 g (74%) of diethyl 4-fluoro-3-(2,6-dimethyl-4-pyridinyl)anilinomethylene-malonate, m.p. 99-100°C. Another 67 g of the crude product was obtained from the mother liquor and was cyclized without further purification as in the following Example 4C to yield 35.2 g of the product of Example 4C (m.p. >300°C.).

C. Ethyl 6-Fluoro-1,4-dihydro-7-(2,6-dimethyl-4-pyridinyl)-4-oxo-3-quinolinecarboxylate - To a 1300 ml of boiling Dowtherm A in a two liter Erlenmeyer flask wrapped with aluminum foil (to maintain the temperature of the Dowtherm A at about 260-265°C.) was added 154 g of diethyl 4-fluoro-3-(2,6-dimethyl-4-pyridinyl)anilinomethylenemalonate over a 5 minute period. The same reaction temperature was maintained for about 20 minutes and then the reaction mixture was allowed to cool. The precipitated tan solid was collected, washed successively with ether and ethanol and dried at 90-95°C. to yield 107.4 g (79%) of ethyl 6-fluoro-1,4-dihydro-7-(2,6-dimethyl-4-pyridinyl)-4-oxo-3-quinolinecarboxylate, m.p. >300°C.

D. Ethyl 1-Ethyl-6-fluoro-1,4-dihydro-7-(2.6-dimethyl-4-pyridinyl)-4-oxo-3-quinolinecarboxylate - A mixture containing 34 g of ethyl 6-fluoro-1,4-dihydro-7-(2,6-dimethyl-4-pyridinyl)-4-oxo-3-quinolinecarboxylate, finely

divided anhydrous potassium carbonate and 250 ml of dimethylformamide was heated with stirring on a steam bath for 20 minutes and then 9 ml of ethyl iodide was added over a 20 minute period. The reaction mixture was heated on a steam bath with stirring for 2 and 1/2 hours and then concentrated to dryness on a rotary evaporator. The residue was shaken well with 300 ml of water and 500 ml of chloroform. The layers were separated and the aqueous layer was extracted with another 200 ml portion of chloroform. The combined chloroform extracts were concentrated *in vacuo* to remove the chloroform. The remaining solid was dissolved in 150 ml of hot isopropyl alcohol, the hot solution treated with decolorizing charcoal and filtered. The filtrate was concentrated on a rotary evaporator to a volume of about 50 ml which was diluted with ether until turbid. The mixture was allowed to cool and the separated crystalline solid was collected, washed with ether and dried at 80-85°C. to yield 18.5 g of ethyl 1-ethyl-6-fluoro-1,4-dihydro-7-(2,6-dimethyl-4-pyridinyl)-4-oxo-3-quinolinecarboxylate, m.p. 221-224°C. Further concentration of the mother liquor yielded another 3.4 g (60%) of product, m.p. 220-223°C. A larger run (0.3 mole scale) gave the above compound, m.p. 222-225°C., in 80% yield.

E. 1-Ethyl-6-fluoro-1,4-dihydro-7-(2,6-dimethyl-4-pyridinyl)-4-oxo-3-quinolinecarboxylic Acid - A mixture containing 98 g of ethyl 1-ethyl-6-fluoro-1,4-dihydro-7-(2,6-dimethyl-4-pyridinyl)-4-oxo-3-quinolinecarboxylate, 50 ml of 35% aqueous sodium hydroxide solution, 500 ml of water and 100 ml of methanol was refluxed with stirring for 90 minutes and the resulting brown solution was acidified with acetic acid whereupon a light yellow precipitate separated. The mixture was allowed to stand at room temperature for about 2 hours and the precipitate was collected, washed successively with water and ethanol and then dried at 90-95°C. to yield 86.8 g (95%) of 1-ethyl-6-fluoro-1,4-dihydro-7-(2,6-dimethyl-4-pyridinyl)-4-oxo-3-quinolinecarboxylic acid, m.p. >310°C.

The usefulness of 1-ethyl-6-fluoro-1,4-dihydro-7-(2,6-dimethyl-4-pyridinyl)-4-oxo-3-quinolinecarboxylic acid (compound of formula I and of Examples 2B and 6E which is designated hereinbelow as Win 52,522) and its uniqueness as a highly potent antibacterial agent having a broad spectrum of antimicrobial activity have been demonstrated by the following experiments.

Experiment 1

COMPARATIVE IN VITRO ANTIBACTERIAL ACTIVITY OF WIN 52,522, WIN 35,439, METHICILLIN AND VANCOMYCIN

As noted above, Win 52,522 is the compound of formula I, i.e., the compound of Example 2B hereinabove. Win 35,439 is its 6-desfluoro analog. Methicillin, used

as its sodium salt (THE MERCK INDEX, 10th Ed., 5842), is 6-(2,6-dimethoxybenzamido)penicillanic acid sodium salt. Vancomycin, used as its hydrochloride (THE MERCK INDEX, 10th Ed., 9731), is an amphoteric glycopeptide antibiotic.

Summary - Win 52,522 (MIC values ≤0.008 to 0.5 mcg/ml) was 2-8 times more potent *in vitro* than Win 35,439 against several Gram-positive cocci, including *S. aureus*, *S. epidermidis* and *Streptococcus* species. Win 52,522 was 32 to >8000 times more potent, and 16 to 125 times more potent, *in vitro* than methicillin and vancomycin, respectively, against the *Staphylococcus* species. Win 52,522 was 2 to 8 times less potent than methicillin against *S. pyogenes* and *S. pneumoniae*, but was 32 to >500 times more potent than methicillin against *S. faecalis*. Win 52,522 was bactericidal (MBC/MIC ≤4) for all 39 bacterial test strains.

Introduction - This study was designed to determine the comparative *in vitro* antibacterial activity of Win 52,522, Win 35,439, methicillin and vancomycin against strains of *Staphylococcus* and *Streptococcus*. *Escherichia coli* and *Pseudomonas aeruginosa* test strains were also examined to illustrate the Gram-negative potency of Win 52,522 relative to that of Win 35,439.

Compound Preparation - Win 52,522 and Win 35,439 were solubilized in 0.1 ml of 0.5 N NaOH/mg of compound and diluted in sterile distilled water to 160 mcg/ml. Vancomycin and methicillin were solubilized in sterile distilled water to 1250 mcq/ml of base compound. The stock solutions were sterilized by filtration through membrane filters with a 0.45 um pore size.

Media

Mueller-Hinton broth (MHB) - BBL

Mueller-Hinton agar (MHA) - BBL

Brain Heart Infusion (BHI) broth - Difco

Brain Heart Infusion (BHI) agar - Difco

Normal horse serum (NHS) - Gibco

Bacterial Cultures - The bacterial cultures listed in Table I (hereinbelow) were grown overnight at 37°C. in MHB for all cultures except the *Streptococcus* strains. These were grown in BHI + 10% NHS. The overnight broth cultures were adjusted to an optical density of 0.10 (650 nm, B L Spectronic 20) in sterile distilled water and subsequently diluted to $6.7 \times 10^6$ cells/ml in MHB or BHI + 1% NHS.

Minimal Inhibitory Concentration (MIC) Test - Serial two-fold dilutions of the compound solutions were prepared in MHB (or in BHI + 1% NHS for tests with Streptococci) and dispensed into 96-well microtiter trays in a volume of 0.1 ml/well using a Sandy Springs (Bellco

Glass Co.) dispenser. The trays were then inoculated with 0.0015 ml of a suspension containing 6.7 x $10^6$ cells/ml of the appropriate inoculum using a MIC-2000 inoculator (Dynatech Corp.) resulting in a final inoculum of approximately 1 x $10^4$ cells/well or 1 x $10^5$ cells/ml. The trays were incubated at 37°C. for 18-20 hours and the minimal inhibitory concentration (MIC), defined as the lowest concentration of compound to inhibit visible bacterial growth, was recorded.

Minimal Bactericidal Concentration (MBC) Test - After the MIC values were recorded, the contents in the microtiter trays were thoroughly mixed on a Bellco Mini-orbital Shaker (Bellco Glass Co.) for 3 min. at a setting of 8. A portion (0.0015 ml) from each well was then transferred via an MIC-2000 inoculator to plates containing MHA or BHI agar + 10% NHS. The plates were incubated at 37°C. for 18-20 hrs. The minimal bactericidal concentration (MBC), defined as the lowest concentration of compound that prevented the formation of a recognizable colony on the agar surface, was recorded.

RESULTS AND DISCUSSION - The individual test results are shown in Table 1 and in summary form in Table 2.

TABLE I: IN VITRO ANTIBACTERIAL ACTIVITY OF WIN 52,522, WIN 35,439, METHICILLIN AND VANCOMYCIN

| Bacteria | WIN 52,522 MIC | WIN 52,522 MBC | Win 35,439 MIC | Win 35,439 MBC | METHICILLIN MIC | METHICILLIN MBC | VANCOMYCIN MIC | VANCOMYCIN MBC |
|---|---|---|---|---|---|---|---|---|
| | MIC and MBC VALUES in mcg/ml | | | | | | | |
| S. aureus 13-8-A | 0.03 | 0.03 | 0.06 | 0.125 | 1 | 2 | 0.5 | 0.5 |
| " " 4-81 | 0.015 | 0.03 | 0.06 | 0.125 | 2 | 2 | 0.5 | 0.5 |
| " " 24-81 | 0.015 | 0.03 | 0.06 | 0.125 | 1 | 2 | 0.5 | 0.5 |
| " " 34-81 | 0.03 | 0.03 | 0.06 | 0.06 | 1 | 2 | 0.5 | 0.5 |
| " " 41-81 | 0.015 | 0.015 | 0.06 | 0.25 | 1 | 1 | 1 | 1 |
| " " 21-81A | 0.015 | 0.015 | 0.06 | 0.125 | 1 | 1 | 0.5 | 1 |
| " " S-234 | 0.015 | 0.03 | 0.06 | 0.125 | 8 | 32 | 1 | 1 |
| " " S-500 | 0.03 | 0.06 | 0.125 | 0.25 | 8 | 32 | 1 | 1 |
| " " 10-81 | 0.03 | 0.03 | 0.125 | 0.125 | 8 | 16 | 1 | 1 |
| " " 59-81 | 0.015 | 0.03 | 0.125 | 0.25 | 32 | 64 | 1 | 1 |
| " " 39881 | 0.015 | 0.03 | 0.06 | 0.06 | 125 | >125 | 1 | 1 |
| " " Smith | 0.03 | 0.03 | 0.125 | 0.125 | 1 | 1 | 1 | 1 |
| " " (25923)* | 0.015 | 0.03 | 0.125 | 0.125 | 2 | 2 | 1 | 1 |
| " " Giorgio | <0.008 | 0.015 | 0.03 | 0.125 | 2 | 4 | 1 | 1 |
| " " VA16951 | 0.015 | 0.015 | 0.06 | 0.06 | 4 | 8 | 1 | 1 |
| S. epidermidis (12228)* | 0.06 | 0.06 | 0.25 | 0.25 | 4 | 4 | 1 | 1 |
| " " (17917)* | 0.06 | 0.125 | 0.5 | 0.5 | 4 | 4 | 1 | 1 |
| (ATCC No. 17917) | | | | | | | | |
| " " 1-3-H | 0.06 | 0.06 | 0.25 | 0.25 | 2 | 4 | 1 | 1 |
| " " 1-5-H | 0.06 | 0.06 | 0.25 | 0.25 | 1 | 2 | 2 | 2 |
| " " 2-1-B | 0.03 | 0.03 | 0.25 | 0.25 | 1 | 2 | 1 | 2 |
| S. pyogenes C203 | 0.5 | 0.5 | 4 | >16 | 0.125 | 0.125 | 16 | 16 |
| " " (89)** | 0.5 | 0.5 | 4 | >16 | ≤0.06 | ≤0.06 | 16 | 16 |
| " " 7948-15 | 0.5 | 0.5 | 4 | >16 | 0.25 | 0.125 | 16 | 16 |
| S. pneumoniae Type I | 0.25 | 0.25 | 2 | 2 | ≤0.06 | ≤0.06 | 16 | 16 |
| " " Type III | 0.5 | 0.5 | 4 | 4 | 0.125 | 0.125 | ≤0.06 | 16 |
| S. faecalis (11700)* | 0.25 | 0.25 | 1 | 1 | 64 | 64 | 1 | >125 |
| " " 4510 | 0.125 | 0.125 | 0.5 | 0.5 | 64 | 64 | 2 | >125 |
| " " (8043)* | 0.25 | 0.5 | 1 | 2 | >125 | >125 | 1 | >125 |
| S. enterococcus 0-2-13 | 0.25 | 0.25 | 2 | 2 | 8 | 8 | 0.25 | 16 |
| E. coli Vogel | 0.25 | 0.5 | 2 | 2 | | | | |
| " " 198 | 0.5 | 0.5 | 2 | 2 | | | | |
| " " (8739)* | 0.25 | 0.25 | 0.5 | 0.5 | | | | |
| " " AH719 | 0.25 | 0.25 | 0.5 | 0.5 | | | | |
| " " 2350 | 0.25 | 0.25 | 0.5 | 0.5 | | | | |
| P. aeruginosa MGH-2 | 16 | 16 | 8 | 16 | | | | |
| " " (7700)* | 16 | 16 | 16 | >16 | | | | |
| " " (15442)* | 16 | 16 | >16 | >16 | | | | |
| " " (9027)* | 4 | 16 | 8 | >16 | | | | |
| " " (27853)* | 16 | 16 | 16 | >16 | | | | |

*ATCC No.
**SWRI No.

TABLE 2: Inhibitory (MIC) and Bactericidal (MBC) Values

| Bacteria (No. Tested) | Compound | MIC (mcg/ml) | | | MBC (mcg/ml | | |
|---|---|---|---|---|---|---|---|
| | | 50% | 90% | Range | 50% | 90% | Range |
| S. aureus (15) | Win 52,522 | 0.015 | 0.03 | ≤0.008-0.03 | 0.03 | 0.03 | 0.015-0.06 |
| | Win 35,439 | 0.06 | 0.125 | 0.03 -0.125 | 0.25 | 0.5 | 0.125-0.5 |
| | Methicillin | 2 | 8 | 1 -125 | 4 | >64 | 2 ->64 |
| | Vancomycin | 1 | 1 | 0.5 -1 | 1 | 1 | 0.5 -1 |
| S. epidermidis (5) | Win 52,522 | 0.06 | 0.06 | 0.03 -0.06 | 0.06 | 0.06 | 0.03 -0.125 |
| | Win 35,439 | 0.25 | 0.25 | 0.25 -0.5 | 0.25 | 0.25 | 0.25 -0.5 |
| | Methicillin | 2 | 4 | 1 -4 | 4 | 4 | 2 -4 |
| | Vancomycin | 1 | 1 | 1 -2 | 1 | 2 | 1 -2 |
| Streptococcus sp. (9) | Win 52,522 | 0.25 | 0.5 | 0.125 -0.5 | 0.5 | 0.5 | 0.125-0.5 |
| | Win 35,439 | 2 | 4 | 0.5 -4 | 2 | >16 | 0.5 ->16 |
| | Methicillin | 0.25 | 64 | ≤0.06 ->125 | 0.125 | 64 | ≤0.06 ->125 |
| | Vancomycin | 2 | 16 | ≤0.06 -16 | 16 | >125 | 16 ->125 |
| E. coli (5) | Win 52,522 | 0.25 | 0.25 | 0.25 -0.5 | 0.25 | 0.5 | 0.25 -0.5 |
| | Win 35,439 | 0.5 | 2 | 0.5 -2 | 0.5 | 2 | 0.5 -2 |
| P. aeruginosa (5) | Win 52,522 | 16 | 16 | 4 -16 | 16 | 16 | 16 |
| | Win 35,439 | 16 | 16 | 8 ->16 | >16 | >16 | 16 ->16 |

From Table 1 it can be seen that Win 52,522 was bactericidal for all the Gram-positive and Gram-negative test strains (MBC/MIC ≤4). Win 35,439 was bacteriostatic against the 3 strains of S. pyogenes. Although less potent than Win 52.522, methicillin was also bactericidal for the Gram-positive cocci, while vancomycin was bacteriostatic for one strain of S. pneumoniae and all 4 strains of S. faecalis.

Relative to Win 35,439, Win 52,522 was 2 to 8 times more potent in vitro against the Gram-positive cocci and E. coli, and similarly potent against P. aeruginosa. These results are consistent with in vivo mouse protection tests where Win 52,522 was 3.6 (s.c.) to 3.9 (p.o.) times more efficacious than Win 35,439 against an S. aureus, and 2.6 (s.c.) to 2.0 (p.o.) times more efficacious against an E coli infection (Experiment 3 hereinbelow).

Win 52,522 was 32 to >8000 times more potent in vitro than methicillin against the Staphylococcus species, 32 to >500 times more potent than methicillin against S. faecalis, but was 2 to 8 times less potent than methicillin against S. pyogenes and S. pneumoniae.

Win 52,522 was 16 to 125 times more potent in vitro than vancomycin against the Gram-positive cocci with two exceptions: 1) Win 52,522 was 8 times less potent than vancomycin against S. pneumoniae type III and, 2) was equal in potency against the one enterococcus (strain 0-2-B) tested.

The *in vitro* test results here support the results from an *in vivo* test showing Win 52,522 to be 16 and >91 times more efficacious than vancomycin and methicillin, respectively, against a methicillin-resistant *Staphylococcus aureus* infection in mice.

From the *in vitro* results reported here and the *in vivo* results shown hereinbelow (Experiment 3), it follows that Win 52,522 should be even more effective than vancomycin in killing deep-seated staphylococcal tissue infections. Vancomycin is an alternate drug of choice for penicillinase-producing *S. aureus* [The Choice of Antimicrobial Drugs, Medical Letter, March 5, 1982] and the drug of choice for the treatment of methicillin resistant *Staphylococcus aureus* [Methicillin-resistant *Staphylococcus aureus*. United States Morbidity and Mortality Weekly Report, November 20, 1981, Vol. 30, No. 45, 557].

## Experiment 2

## ANTIMICROBIAL ACTIVITY OF WIN 52,522, WIN 35,439, ROSOXACIN, NORFLOXACIN, PEFLOXACIN AND TETRACYCLINE AGAINST ANAEROBIC AND MICROAEROPHILIC BACTERIA ASSOCIATED WITH ORAL INFECTIONS

Summary - Win 52,522 was found to have high activity against all thirteen of the target anaerobic microorganisms and compared favorably with tetracycline, the current antibiotic of choice.

Introduction - It is generally accepted that certain microorganisms are the primary etiologic agents in the initiation of periodontal diseases [Socransky et al., Present Status of Studies on the Microbial Etiology of Periondontal Diseases, pp. 1-14. In: R. Genco and S. Mergenhagen (Ed.), Host-Parasite Interactions in Periodontal Diseases, 1982, Amer. Soc. Micro. Washington, DC.] Furthermore, there is increasing evidence to support the hypothesis that specific microorganisms are associated with each of the various forms of periodontal disease [Socransky et al., supra]. Accordingly, the rationale has been advanced for the use of antimicrobials in the prevention, control and treatment of these pandemic diseases. There is evidence in various animal models that the administration of various antibiotics can reduce the clinical signs of gingivitis and also decrease the progression of alveolar bone loss associated with aggressive periodontal diseases [Gibson, Antibiotics and Periodontal Disease: A Selective Review, J. Amer. Dent. Assoc. 104, 213-218 (1982)]. In man, the data would also appear supportive of the argument that antimicrobial agents are extremely useful in treating established disease, particularly when used to augment conventional therapies [Gibson, supra]. In this regard, the antibiotics tetracycline and metronidazole have been shown to be particularly useful [Genco, Antibiotics in the Treatment of Human Periodotal Diseases. J. Periodontol. 52, 545-558 (1981)]; however, these agents do not appear to be ideal since they are not active against all the microorganisms considered to participate in the disease process. Furthermore, there are an increasing number of reports indicating that many of the so-called primary pathogens of periodontal disease have already evidenced patterns of increased tolerance to these agents [Gibson, supra, Genco, supra and Williams et al., Subgingival Microflora of Periodontal Patients On Tetracycline Therapy, J. Clin. Periodontol. 6, 210-215 (1979)]. Hence, there exists an important and timely need to discover and develop new antimicrobial agents which will have utility in the prevention/management of periodontal diseases. The studies described herein show that Win 52,522 was found to be extremely active against a number of anaerobic microorganisms associated with periodontal diseases.

Microorganisms - The microorganisms used in these studies are listed in Table A hereinbelow.

Culture Conditions

1. Maintenance. Prior to use, all strains were maintained in the lyophilized state. During the study, cultures were examined on blood agar basal medium supplemented with 5.0% (v/v) defibrinated rabbit blood. Culture transfer was accomplished weekly with all cultures being incubated in an anaerobic atmosphere (GasPak jar; BBL).

2. Agar Medium. The basal test medium consisted of trypticase soy agar (40 g), yeast extract (1 g), glucose (2 g) and 960 ml of distilled water pH 7.2. [Mashimo et al., J. Clin. Micro. 17, 187-191 (1983)]. Subsequent to sterilization and cooling to 50°C., the medium was supplemented with 4% (v/v) defibrinated rabbit blood and 0.5 μg/ml menadione (Vitamin $K_3$). The agent under evaluation was also added at this time to the concentration desired. Subsequent to pouring of the plates, they were permitted to dry at 4°C. for 24 hr.

3. Determination of Agent MIC. The minimal inhibitory concentration (MIC) of the various test agents was determined using an agar dilution technique. Agents and appropriate reference compounds were added to achieve a series of concentrations ranging between 32 μg/ml and 0.007 μg/ml. The inocula were prepared from 96-hr. cultures grown on the maintenance medium. Subsequent to a purity evaluation, colonies were removed and suspended in trypticase soy broth supplemented with 5 μg/ml menadione and 2% lysed rabbit blood filtrate to achieve an $OD_{650}$ of 0.1 (this corresponded to approximately $10^8$ cells/ml). Cultures were inoculated aerobically on the modified trypticase soy blood agar plates by use of a Steer's replicator (which delivers about $10^5$ cells per spot). The plates were allowed to dry for a time not exceeding 30 min. and then incubated at 37°C. under an

atmosphere of 80% $N_2$, 10% $H_2$ and 5% $CO_2$ maintained in an anaerobic chamber (Coy Manufacturing Co.) for 48 hr. The MIC was defined as the lowest concentration completely inhibiting growth.

Agents - Tetracycline stock solutions (1 mg/ml) were made subsequent to dissolution in water. Stock solutions (1 mg/ml) of the remaining test compounds were made subsequent to dissolution in 1/10 N NaOH. Appropriate controls indicated at the levels used NaOH did not affect the viability of the test microorganisms.

Results - The antimicrobial activities of the compounds tested are shown in Table A.

TABLE A SUSCEPTIBILITY OF SELECTED ANAEROBIC MICROORGANISMS TO TEST COMPOUNDS

| MICROORGANISMS | Win Number µg/ml 35,213[a] | 35,439[b] | 52,522[c] | 9,424[d] | 50,297[e] | 50,150[f] |
|---|---|---|---|---|---|---|
| F. polymorphum | >10 | 4 | 0.5 | 0.12 | >10 | 8 |
| A. viscosus | >10 | 8 | 1.0 | 2.0 | >10 | >10 |
| B. melaninogenicus | 8 | 8 | 1.0 | 0.5 | 4.0 | 4 |
| Capnocytophaga suptigena | 1 | 0.5 | 0.06 | 1.0 | 0.5 | 0.25 |
| B. intermedius | 0.5 | 0.25 | 0.06 | 2.0 | 0.5 | 0.5 |
| Eikenella corrodens | 8 | 2.0 | 0.5 | 2.0 | 4.0 | 4.0 |
| B. oralis | >10 | >10 | 4.0 | 0.12 | 4.0 | 4.0 |
| B. gingivalis | >10 | >10 | 2.0 | 0.12 | 4.0 | 4.0 |
| A. actinomycetemcomitans | 0.12 | 0.25 | 0.12 | 1.0 | 0.06 | 0.06 |
| B. fragilis | >10 | >10 | 4.0 | 1.0 | >10 | 8.00 |
| F. nucleatum | >10 | 4 | 0.5 | 0.75 | 8.00 | 8.00 |
| Wolinella recta | 0.5 | 0.5 | 0.12 | - | 0.12 | 0.25 |
| Campylobacter sputorum | >10 | >10 | 8.0 | - | 2.0 | 4.0 |

a) Rosoxacin
b) 6-Desfluoro analog of Win 52,522
c) Applicants' Example 2B
d) Tetracycline
e) Norfloxacin
f) Pefloxacin

Win 52,522 is seen to be markedly superior to both Win 35,213 and 35,439. In fact, Win 52,522 showed a broad spectrum of antimicrobial activity, comparing favorably with the currant antibiotic of choice, tetracycline. Wins 50,297 and 50,150 were less active.

Experiment 3

IN VIVO ANTIBACTERIAL ACTIVITY OF WIN 52,522 COMPARED TO WIN 35,439 AGAINST STAPHYLOCOCCUS AUREUS AND ESCHERICHIA COLI INFECTIONS IN MICE

SUMMARY - Win 52,522 was determined to be more efficacious than Win 35,439, by either subcutaneous and oral routes of medication, against infections in mice produced by penicillin-sensitive and methicillin-resistant *Staphylococcus aureus strains*.

MATERIALS AND METHODS

Animals - ICR mice, female, 18-20 grams each.

Antibacterial Agents - Win 52,522, Win 35,439, methicillin (Win 9,410) and vancomycin (Win 39,556).

Bacterial Cultures

*Escherichia coli* Vogel

*Staphylococcus aureus* 39881

*Staphylococcus aureus* Smith

Culture Media

Brain Heart Infusion Agar (Difco)

Brain Heart Infusion Broth (Difco)

0179239

Gastric Mucin - Bacteriological grade, Lot No. 0617, ICN Pharmaceuticals, Inc. A 5% (w/v) suspension was prepared by adding 25 grams of gastric mucin to 475 ml of cold tap water. The suspension was mixed for 20-30 minutes with an Omni-mixer, stored at 4°C. overnight and just before use brought to pH 7.2 with 40% (w/v) NaOH.

Preparation of Antimicrobial Drug Solutions - Solutions of Win 52,522 and Win 35,439 were prepared by dissolving the appropriate weight of drug in 0.3 to 0.4 ml of 1 N NaOH and then bringing the solutions to the desired volume with distilled water. Methicillin and vancomycin were dissolved directly in distilled water.

Preparation of Bacterial Cultures

A. Staphylococcus aureus Smith - Two and one-half ml of pool were added to 247.5 ml of 5% gastric mucin to provide the test inoculum containing 2.1 x $10^4$ cells/ml.

B. Staphylococcus aureus 39881 - The culture was streaked onto three Brain Heart Infusion Agar plates and incubated 16 hours at 37°C. The cells were washed off each plate with five ml of saline and the cell suspension was shaken with glass beads for two minutes on a paint shaker. The suspension was diluted with saline so that a 1:00 dilution gave a 44% T reading on a B + L Spectronic 20 (650 nm) calibrated with saline. The stock suspension was diluted 1:24 (10 ml stock suspension + 230 ml 5% mucin) to obtain the test inoculum containing 1.55 x $10^9$ cells/ml.

C. Escherichia coli Vogel - The culture was inoculated into 10 ml of Brain Heart Infusion broth and grown statically for 16 hours at 37°C. Fresh tubes of the same broth were inoculated with 0.1 ml portions of the 16 hour broth culture and incubation continued for 5 hours at 37°C. The 5 hour broth culture was finally diluted with saline to provide the test inoculum containing $4.0 \times 10^7$ cells/ml.

Infection of Mice - Mice were inoculated intraperitoneally with 0.5 ml of the bacterial test inoculum.

Medication of Mice

A. Staphylococcus aureus Smith

1. Single dose test - Mice were medicated subcutaneously or orally once (0.5 ml) one-half hour postinfection.

2. Multiple dose test - Mice were medicated subcutaneously (0.2 ml) or orally (0.5 ml) at the following times: six hours and one hour preinfection (infection at 2 pm) and twice a day (8 a.m. and 3:30 p.m.) for the next two successive days.

B. Staphylococcus aureus 39881 - Mice were medicated twice: at one-half and four hours postinfection. The drugs were administered by both subcutaneous (0.2 ml) and oral (0.5 ml) routes.

C. Escherichia coli Vogel - Mice were medicated subcutaneously or orally once (0.5 ml) one-half hour postinfection.

Deaths were recorded daily for seven days for all tests. Fifty percent protective dose values ($PD_{50}$'s) were calculated using probit analysis [SAS Users Guide: Statistics, 1982 Edition, SAS Institute, Inc., Cary, North Carolina].

## RESULTS

The *in vivo* antibacterial activity of Win 52,522, as compared to Win 35,439, against a *Staphylococcus aureus* Smith infection in mice is shown in Tables 1 and 2.

TABLE 1

In Vivo Antibacterial Activity of Win 52,522 and Win 35,439 Against a Staphylococcus aureus Smith Infection in Mice - Single Dose Test

| Drug | Dose (mg/kg) | Route | Survivors/ Total | Protective Dose 50% (mg/kg) (95% Confidence Limits) |
|---|---|---|---|---|
| Win 52,522 | 0.095 | sc | 0/10 | 0.8(0.6-1.1) |
| | 0.19 | | 0/10 | |
| | 0.39 | | 2/10 | |
| | 0.78 | | 4/10 | |
| | 1.56 | | 10/10 | |
| Win 35,439 | 0.78 | sc | 1/10 | 2.9(2.3-4.3) |
| | 1.56 | | 0/10 | |
| | 3.13 | | 6/10 | |
| | 6.25 | | 10/10 | |
| Win 52,522 | 0.19 | po | 0/10 | 1.4* |
| | 0.39 | | 0/10 | |
| | 0.78 | | 0/10 | |
| | 1.56 | | 7/10 | |
| | 3.13 | | 10/10 | |
| Win 35,439 | 0.39 | po | 0/10 | 5.5* |
| | 0.78 | | 1/10 | |
| | 1.56 | | 0/10 | |
| | 3.13 | | 0/10 | |
| | 6.25 | | 7/10 | |
| Vancomycin | 0.78 | sc | 2/10 | 1.1(0.6-1.5) |
| | 1.56 | | 9/10 | |
| | 3.13 | | 9/10 | |
| | 6.25 | | 10/10 | |
| | 12.5 | | 10/10 | |
| Infected Controls | | - | 0/20 | |

*Estimate

Table 2

0179239

*In Vivo* Antibacterial Activity of Win 52,522 and Win 35,439 Against a *Staphylococcus aureus* Smith Infection in Mice - Multiple Dose Test

| Drug | Dose (mg/kg) | Route | Survivors/ Total | Protective Dose 50% (mg/kg) (95% Confidence Limits) |
|---|---|---|---|---|
| Win 52,522 | 0.19 | sc | 0/10 | 1.8(1.3-2.4) |
| | 0.39 | | 0/10 | |
| | 0.78 | | 0/10 | |
| | 1.56 | | 4/10 | |
| | 3.13 | | 9/10 | |
| | 6.25 | | 10/10 | |
| | 12.5 | | 10/10 | |
| Win 35,439 | 3.13 | sc | 1/10 | 7.1(5.4-10.2) |
| | 6.25 | | 3/10 | |
| | 12.5 | | 10/10 | |
| | 25 | | 10/10 | |
| Win 52,522 | 0.19 | po | 0/10 | 2.1(1.6-2.7) |
| | 0.39 | | 0/10 | |
| | 0.78 | | 0/10 | |
| | 1.56 | | 2/10 | |
| | 3.13 | | 9/10 | |
| | 6.25 | | 10/10 | |
| | 12.5 | | 10/10 | |
| Win 35,439 | 3.13 | po | 0/10 | 7.2(5.4-9.6) |
| | 6.25 | | 4/10 | |
| | 12.5 | | 9/10 | |
| | 25 | | 10/10 | |
| Infected Controls | - | - | 0/20 | |

Win 52,522 was 3.6 (sc) and 3.9 (po) times more efficacious than Win 35,439 when administered as a single dose one-half hour postinfection. Win 52,522 was 3.9 (sc) and 3.4 (po) times more efficious than Win 35,439 against the same infection when given in a multiple dose regimen.

$PD_{50}$ values with Win 52,522 against a methicillin-resistant Staphylococcus aureus infection in mice were 1.1 and 1.6 mg/kg for subcutaneous and oral medication, respectively, as shown in Table 3.

Table 3

In Vivo Antibacterial Activity of Win 52,522, Win 35,439, Methicillin and Vancomycin Against a Staphylococcus aureus 39881 (Methicillin-resistant) Infection in Mice

| Drug | Dose** (mg/kg) | Route | Survivors/ Total | Protective Dose 50% (mg/kg) (95% Confidence Limits) |
|---|---|---|---|---|
| Win 52,522 | 0.19 | sc | 0/10 | 1.1(0.1-4.1) |
| | 0.39 | | 0/10 | |
| | 0.78 | | 2/10 | |
| | 1.56 | | 10/10 | |
| | 3.13 | | 9/10 | |
| | 6.25 | | 10/10 | |
| Win 35,439 | 1.56 | sc | 0/10 | 4.9* |
| | 3.13 | | 0/10 | |
| | 6.25 | | 9/10 | |
| | 12.5 | | 7/10 | |
| Win 52,522 | 0.19 | po | 0/10 | 1.6(1.3-2.4) |
| | 0.39 | | 0/10 | |
| | 0.78 | | 1/10 | |
| | 1.56 | | 4/10 | |
| | 3.13 | | 10/10 | |
| | 6.25 | | 10/10 | |
| Win 35,439 | 1.56 | po | 1/10 | 7.2(5.6-10.0) |
| | 3.13 | | 0/10 | |
| | 6.25 | | 3/10 | |
| | 12.5 | | 10/10 | |
| Methicillin | 25 | sc | 0/10 | >100 |
| | 50 | | 0/10 | |
| | 100 | | 0/10 | |
| Vancomycin | 6.25 | sc | 0/10 | 17.7(13.8-22.6) |
| | 12.5 | | 1/10 | |
| | 25 | | 9/10 | |
| | 50 | | 10/10 | |
| Infected Controls | - | - | 0/20 | |

*Estimate

**Two doses - one-half and four hours postinfection

Win 52,522 (sc) was 4.5 and 16 times more active against that infection than Win 35,439 and vancomycin, respectively.

The *in vivo* activity of Win 52,522 and 35,439 against an *Escherichia coli* Vogel infection in mice is shown in Table 4.

Table 4

*In Vivo* Antibacterial Activity of Win 52,522 and Win 35,439 Against a *Escherichia coli* Vogel Infection in Mice

| Drug | Dose (mg/kg) | Route | Survivors/ Total | Protective Dose 50% (mg/kg) (95% Confidence Limits) |
|---|---|---|---|---|
| Win 52,522 | 1.56 | sc | 0/10 | 5.2* |
| | 3.13 | | 0/10 | |
| | 6.25 | | 8/10 | |
| | 12.5 | | 10/10 | |
| | 25 | | 10/10 | |
| Win 35,439 | 3.13 | sc | 1/10 | 13.5(10.5-19.2) |
| | 6.25 | | 0/10 | |
| | 12.5 | | 4/10 | |
| | 25 | | 10/10 | |
| Win 52,522 | 1.56 | po | 0/10 | 8.2(6.3-10.8) |
| | 3.13 | | 0/10 | |
| | 6.25 | | 2/10 | |
| | 12.5 | | 9/10 | |
| | 25 | | 10/10 | |
| Win 35,439 | 3.13 | po | 0/10 | 16.5(12.6-21.8) |
| | 6.25 | | 0/10 | |
| | 12.5 | | 2/10 | |
| | 25 | | 9/10 | |
| Infected Controls | - | - | 0/20 | |

*Estimate

Subcutaneous and oral $PD_{50}$ values of Win 52,522 against that infection were 5.2 and 8.2 mg/kg., approximately one-half those of Win 35,439.

The results of this studv demonstrate that Win 52,522, by both parenteral and oral medication routes, is a very potent antibacterial agent against infections in mice produced by both penicillin-sensitive and methicillin-resistant Staphylococcus aureus strains.

The compound of formula I or a pharmaceutically-acceptable non-toxic acid-addition or cationic salt thereof can be prepared for use by conventional pharmaceutical procedures: that is, bv dissolving or suspending them in a pharmaceutically acceptable vehicle, e.g., water, aqueous alcohol; glycol, oil solution or oil-water emulsion, for parenteral or oral administration; or by incorporating them in unit dosage form as capsules or tablets for oral administration either alone or in combination with conventional adjuvants or excipients, e.g., calcium carbonate, starch, lactose, talc, magnesium stearate, gum acacia, and the like.

## CLAIMS

1. 1-Ethyl-6-fluoro-1,4-dihydro-7-(2,6-dimethyl-4-pyridinyl)-4-oxo-3-quinolinecarboxylic acid of the formula

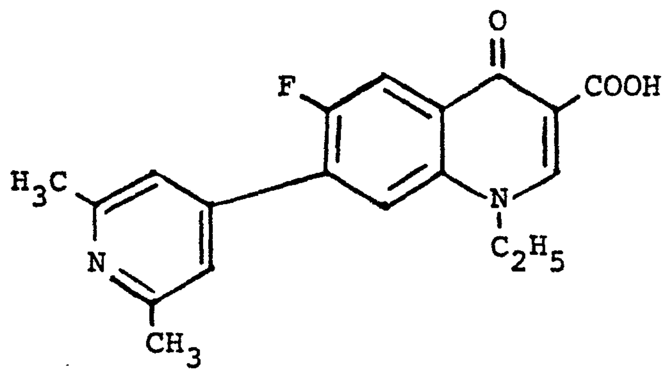

or a pharmaceutically acceptable, non-toxic acid-addition or cationic salt thereof.

2. A process for preparing a compound according to claim 1, which comprises

a. converting 6-amino-1-ethyl-1,4-dihydro-7-(2,6-dimethyl-4-pyridinyl)-4-oxo-3-quinolinecarboxylic acid via its 6-diazonium salt to produce 1-ethyl-6-fluoro-1,4-dihydro-7-(2,6-dimethyl-4-pyridinyl)-4-oxo-3-quinolinecarboxylic acid or

b. ethylating a 6-fluoro-1,4-dihydro-7-(2,6-dimethyl-4-pyridinyl)-4-oxo-3-quinolinecarboxylic acid or lower-alkyl ester thereof and in the case of the reaction with the lower alkyl ester thereby producing a lower-alkyl ester of 1-ethyl-6-fluoro-1,4-dihydro-7-(2,6-dimethyl-4-pyridinyl)-4-oxo-3-quinolinecarboxylic acid, hydrolyzing said ester to produce the corresponding acid,

and, if desired, converting a free base obtained to an acid-addition salt thereof or converting a compound obtained to a cationic salt thereof by reaction with a strong inorganic or organic base.

3. A process according to claim 2, in which in a., the 6-amino-1-ethyl-1,4-dihydro-7-(2,6-dimethyl-4-pyridinyl)-4-oxo-3-quinolinecarboxylic acid is prepared by nitrating

1-ethyl-1,4-dihydro-7-(2,6-dimethyl-4-pyridinyl)-4-oxo-3-quinolinecarboxylic acid to produce 1-ethyl-1,4-dihydro-7-(2,6-dimethyl-4-pyridinyl)-6-nitro-4-oxo-3-quinolinecarboxylic acid, and reducing said 6-nitro compound.

4. A process according to claim 2, in which in b., the 6-fluoro-1,4-dihydro-7-(2,6-dimethyl-4-pyridinyl)-4-oxo-3-quinolinecarboxylic acid is prepared by reducing 4-(2-fluoro-5-nitrophenyl)-2,6-dimethylpyridine to produce 4-(5-amino-2-fluorophenyl)-2,6-dimethylpyridine, reacting the latter compound of (Example 6A) with a di-lower alkyl ethoxymethylenemalonate to produce a di-lower-alkyl 3-(2,6-dimethyl-4-pyridinyl)-4-fluoroanilinomethylenemalonate and refluxing the latter compound to produce a lower-alkyl 6-fluoro-1,4-dihydro-7-(2,6-dimethyl-4-pyridinyl)-4-oxo-3-quinolinecarboxylate, and, if the carboxylic acid is desired, hydrolyzing said lower-alkyl carboxylate thus obtained.

5. A process for purifying 1-ethyl-6-fluoro-1,4-dihydro-7-(2,6-dimethyl-4-pyridinyl)-4-oxo-3-quinolinecarboxylic acid containing as impurities the corresponding 6-desfluoro and 6-hydroxy compounds by converting it to its corresponding lower-alkyl 1-ethyl-6-fluoro-1,4-dihydro-7-(2,6-dimethyl-4-pyridinyl)-4-oxo-3-carboxylate, separating the ester from the reaction mixture and saponifying the ester to produce the purified acid.

6. A compound of the formula

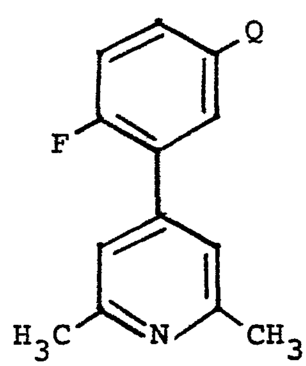

where Q is hydrogen or nitro, or an acid-addition salt thereof.

7. A process for preparing a compound according to

claim 6, which comprises heating 4-(2-fluorophenyl)-2,6-dimethyl-3,5-pyridinecarboxylic acid to produce a mixture of 4-(2-fluorophenyl)-2,6-dimethylpyridine and 2,4-dimethyl-5H-[1]benzopyrono[3,4-c]pyridin-5-one, separating the components of the mixture to isolate the 4-(2-fluorophenyl)-2,6-dimethylpyridine and, if desired, nitrating the latter compound to produce 4-(2-fluoro-5-nitrophenyl)-2,6-dimethylpyridine.

8. A process for producing 4-(2-fluoro-5-nitrophenyl)-2,6-dimethylpyridine which comprises nitrating 4-(2-fluorophenyl)-2,6-dimethylpyridine.

9. A composition for combatting bacteria which comprises an antibacterially effective amount of a compound according to claim 1 together with one or more pharmaceutically acceptable vehicles or excipients.

10. A method for combatting bacteria, which comprises contacting the locus of said bacteria with a compound according to claim 1.

CLAIMS AT



1. A process for preparing a 4-oxo-3-quinolinecarboxylic acid of the formula

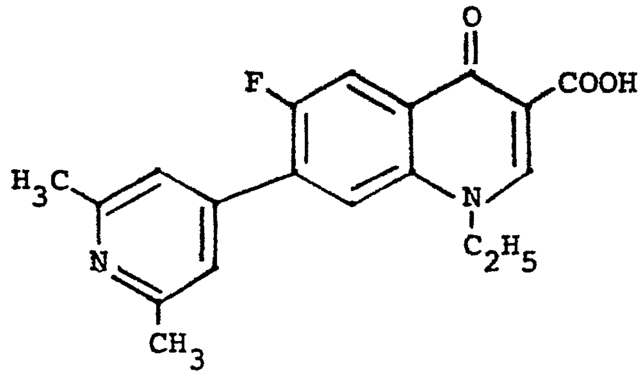

or a pharmaceutically acceptable, non-toxic acid-addition or cationic salt thereof, characterized by

a. converting 6-amino-1-ethyl-1,4-dihydro-7-(2,6-dimethyl-4-pyridinyl)-4-oxo-3-quinolinecarboxylic acid via its 6-diazonium salt to produce 1-ethyl-6-fluoro-1,4-dihydro-7-(2,6-dimethyl-4-pyridinyl)-4-oxo-3-quinolinecarboxylic acid or

b. ethylating a 6-fluoro-1,4-dihydro-7-(2,6-dimethyl-4-pyridinyl)-4-oxo-3-quinolinecarboxylic acid or lower-alkyl ester thereof and in the case of the reaction with the lower alkyl ester thereby producing a lower-alkyl ester of 1-ethyl-6-fluoro-1,4-dihydro-7-(2,6-dimethyl-4-pyridinyl)-4-oxo-3-quinolinecarboxylic acid, hydrolyzing said ester to produce the corresponding acid,

and, if desired, converting a free base obtained to an acid-addition salt thereof or converting a compound obtained to a cationic salt thereof by reaction with a strong inorganic or organic base.

2. A process according to claim 1, in which in a., the 6-amino-1-ethyl-1,4-dihydro-7-(2,6-dimethyl-4-pyridinyl)-4-oxo-3-quinolinecarboxylic acid is prepared by nitrating 1-ethyl-1,4-dihydro-7-(2,6-dimethyl-4-pyridinyl)-4-oxo-3-quinolinecarboxylic acid to produce 1-ethyl-1,4-dihydro-7-(2,6-dimethyl-4-pyridinyl)-6-nitro-4-oxo-3-quinolinecarboxylic acid, and reducing said 6-nitro compound.

CASE: 3744-59

3. A process according to claim 1, in which in b., the 6-fluoro-1,4-dihydro-7-(2,6-dimethyl-4-pyridinyl)-4-oxo-3-quinolinecarboxylic acid is prepared by reducing 4-(2-fluoro-5-nitrophenyl)-2,6-dimethylpyridine to produce 4-(5-amino-2-fluorophenyl)-2,6-dimethylpyridine, reacting the latter compound of (Example 6A) with a di-lower alkyl ethoxymethylenemalonate to produce a di-lower-alkyl 3-(2,6-dimethyl-4-pyridinyl)-4-fluoroanilinomethylenemalonate and refluxing the latter compound to produce a lower-alkyl 6-fluoro-1,4-dihydro-7-(2,6-dimethyl-4-pyridinyl)-4-oxo-3-quinolinecarboxylate, and, if the carboxylic acid is desired, hydrolyzing said lower-alkyl carboxylate thus obtained.

4. A process for purifying 1-ethyl-6-fluoro-1,4-dihydro-7-(2,6-dimethyl-4-pyridinyl)-4-oxo-3-quinolinecarboxylic acid containing as impurities the corresponding 6-desfluoro and 6-hydroxy compounds characterized by converting it to its corresponding lower-alkyl 1-ethyl-6-fluoro-1,4-dihydro-7-(2,6-dimethyl-4-pyridinyl)-4-oxo-3-carboxylate, separating the ester from the reaction mixture and saponifying the ester to produce the purified acid.

5. A process according to claim 4, where lower-alkyl is ethyl.

6. A process for preparing a compound of the formula

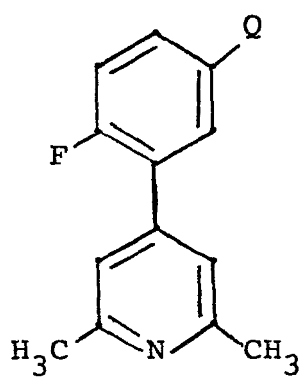

where Q is hydrogen or nitro, or an acid-addition salt thereof, characterized by heating 4-(2-fluorophenyl)-2,6-dimethyl-3,5-pyridinecarboxylic acid to produce a mixture of 4-(2-fluorophenyl)-2,6-dimethylpyridine and 2,4-dimethyl-5H-[1]benzopyrono[3,4-c]pyridin-5-one, separating the components of the mixture to isolate the 4-(2-fluorophenyl)-2,6-dimethylpyridine and, if desired, nitrating the latter compound to produce 4-(2-fluoro-5-nitrophenyl)-2,6-dimethylpyridine.

7. A process for producing 4-(2-fluoro-5-nitrophenyl)-2,6-dimethylpyridine characterized by nitrating 4-(2-fluorophenyl)-2,6-dimethylpyridine.